# EUROPEAN PATENT APPLICATION

(11) **EP 4 502 161 A1**
(43) Date of publication of application: **05.02.2025**
(21) Application number: 23780946.2
(22) Date of filing: 30.03.2023
(51) Int. Cl.: C12N 15/56, C11D 3/386, C12N 1/15, C12N 1/19, C12N 1/21, C12N 5/10, C12N 9/28, C12N 15/31, C12N 15/63

(54) **ALPHA-AMYLASE VARIANT**

(30) Priority: 31.03.2022 JP 2022060424
(71) Applicant: Kao Corporation, Tokyo 103-8210 (JP)
(72) Inventor: SHAKU, Mao, Wakayama-shi, Wakayama 640-8580 (JP); HIOKI, Takahiro, Wakayama-shi, Wakayama 640-8580 (JP); KAWAHARA, Akihito, Wakayama-shi, Wakayama 640-8580 (JP)
(74) Representative: Vossius & Partner Patentanwälte Rechtsanwälte mbB
(86) International application number: PCT/JP2023/013317
(87) International publication number: WO 2023/190938

(57) **Abstract**

Provided is an α-amylase which has excellent amylolytic activity at low temperatures and further has excellent stability at low pHs and/or in the presence of a chelating agent. α-Amylase mutants A1, B1, C1, D1, and E1 derived from the following parent amylases: an α-amylase A having at least 90% sequence identity to the amino acid sequence of SEQ ID NO: 4; an α-amylase B having at least 90% sequence identity to the amino acid sequence of SEQ ID NO: 6; an α-amylase C having at least 90% sequence identity to the amino acid sequence of SEQ ID NO: 8; an α-amylase D having at least 90% sequence identity to the amino acid sequence of SEQ ID NO: 10; and an α-amylase E having at least 90% sequence identity to the amino acid sequence of SEQ ID NO: 12, respectively, wherein the mutants A1 to E1 each comprises deletion of amino acid residues at two positions selected from the group consisting of positions corresponding to positions 178 to 181 according to the numbering of SEQ ID NO: 1, and substitution of amino acid residues at three or more positions selected from the group consisting of positions corresponding to positions 178, 181, 238, 239, 240, 126, 129, and 201 according to the numbering of SEQ ID NO: 2, the substitution including at least the following a) to e): a) in A1, 178H, 181E or 181Q, and 239Q; b) in B1, 178H, 181E or 181Q, and 239Q; c) in C1, 178H, 181E or 181Q, 238F, and 239Q; d) in D1, substitution at three or more positions selected from the group consisting of 178H, 181E or 181Q, 238F, and 239Q; and e) in E1, substitution at three or more positions selected from the group consisting of 178H, 181E or 181Q, 238F, 239Q, and 240F.

## Description

### Field of the Invention

The present invention relates to an α-amylase mutant.

### Background of the Invention

α-Amylases are used in a wide range of industries, such as starch, brewing, textiles, pharmaceuticals, and food, are known to have suitability for incorporation into cleaning agents, and are incorporated into dishwashing cleaning agents for automatic dishwashers and clothing cleaning agents as components removing starch stains.

Known α-amylases useful for cleaning agents are *Bacillus* sp. KSM-1378 (FERM BP-3048) strain-derived α-amylase AP1378 (Patent Literature 1), Termamyl and Duramyl (registered trademarks), which are *Bacillus licheniformis*-derived α-amylases, *Bacillus* sp. DSM12649 strain-derived α-amylase AA560 (Patent Literature 2), *Bacillus* sp. SP722 strain-derived α-amylase SP722 (SEQ ID NO: 4 of Patent Literature 3), Cytophaga-derived α-amylase CspAmy2 (Patent Literature 4), LABM, which is a chimera of the A and B domains of *Bacillus-derived* α-amylase AAI10 and *Alicyclobacillus*-derived α-amylase (SEQ ID NO: 13 of Patent Literature 5), and the like. With regard to these α-amylases, mutants modified to improve their functions for specific applications, for example, mutants with enhanced stability in cleaning agents, have been reported (Patent Literature 6).

However, if the parent enzymes are different, the same effect will not necessarily be obtained by mutation. In particular, obtaining multiple mutants with significantly enhanced specific properties requires a great deal of effort to tailor each parent enzyme.

On the other hand, in recent years, it has been known that since cleaning in a low-pH environment is gentle on items to be cleaned and is not as strong as commonly used alkaline compositions, it has an effect to keep items to be cleaned, such as glass and patterned dishes, looking new for longer, reduces uncomfortable odors on fabrics, aids in the release of calcium soaps which tend to trap stains on fabrics, improves performance against pH-sensitive stains, and further benefits the feel of fabrics. In addition, dishwashing cleaning agents are not only used to clean dishes, which are main cleaning targets, but also used to clean stainless steel and plastic sinks, so that they may be incorporated with high concentrations of chelating agents, such as citric acid, which have detergency against limescale stains. Therefore, it can be expected that the addition of enzymes, such as α-amylases, to such cleaning agents will further improve their detergency.

Accordingly, the α-amylases for cleaning agents described above are also required to have enhanced stability in acidic or chelating agent-containing cleaning agents, particularly acidic and chelating agent-containing cleaning agents.

[Patent Literature 1] WO 94/26881
[Patent Literature 2] WO 00/60060
[Patent Literature 3] WO 06/002643
[Patent Literature 4] WO 2014/164777
[Patent Literature 5] JP-A-2018-516553
[Patent Literature 6] WO 98/044126

### Summary of the Invention

The present invention relates to the following:
1) α-Amylase mutants A1, B1, C1, D1, and E1 derived from the following parent amylases: an α-amylase A having at least 90% sequence identity to the amino acid sequence of SEQ ID NO: 4; an α-amylase B having at least 90% sequence identity to the amino acid sequence of SEQ ID NO: 6; an α-amylase C having at least 90% sequence identity to the amino acid sequence of SEQ ID NO: 8; an α-amylase D having at least 90% sequence identity to the amino acid sequence of SEQ ID NO: 10; and an α-amylase E having at least 90% sequence identity to the amino acid sequence of SEQ ID NO: 12, respectively, wherein the mutants A1 to E1 each comprises deletion of amino acid residues at two positions selected from the group consisting of positions corresponding to positions 178 to 181 according to the numbering of SEQ ID NO: 1, and substitution of amino acid residues at three or more positions selected from the group consisting of positions corresponding to positions 178, 181, 238, 239, 240, 126, 129, and 201 according to the numbering of SEQ ID NO: 2, the substitution comprising at least the following a) to e) :
   a) in A1, substitution of an amino acid residue at a position corresponding to position 178 with H, substitution of an amino acid residue at a position corresponding to position 181 with E or Q, and substitution of an amino acid residue at a position corresponding to position 239 with Q;
   b) in B1, substitution of an amino acid residue at a position corresponding to position 178 with H, substitution of an amino acid residue at a position corresponding to position 181 with E or Q, and substitution of an amino acid residue at a position corresponding to position 239 with Q;
   c) in C1, substitution of amino acid residues at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q;
   d) in D1, substitution of amino acid residues at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q; and
   e) in E1, substitution of amino acid residues at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F.
2) A polynucleotide encoding the mutant according to 1) .
3) A vector or DNA fragment comprising the polynucleotide according to 2).
4) A transformed cell comprising the vector or DNA fragment according to 3).
5) A cleaning composition comprising the mutant according to 1).

### Brief Description of Drawings

[Fig. 1] Fig. 1 shows the alignment of SEQ ID NO: 1, SEQ ID NO: 2, and five parent α-amylases.
[Fig. 2] Fig. 2 shows the alignment of SEQ ID NO: 2 and five α-amylases with a deletion of two amino acids.

### Detailed Description of the Invention

The present invention relates to the provision of an α-amylase which exhibits excellent stability at low pHs and/or in the presence of a chelating agent.

The present inventors found that common specific amino acid modifications of various amylases for cleaning agents result in a remarkable stabilizing effect at low pHs and/or in the presence of a chelating agent.

The present invention can provide an α-amylase mutant having enhanced stability at low pHs and/or in the presence of a chelating agent, in comparison with a parent α-amylase. The use of such an α-amylase mutant allows cleaning stably exhibiting a starch stain removal effect even at low pHs, further in the presence of a chelating agent.

In the present specification, "amylase" (EC3.2.1.1; α-D-(1→4)-glucan glucanohydrolase) refers to a group of enzymes which catalyze the hydrolysis of starch and other linear or branched 1,4-glycoside oligosaccharides or polysaccharides. The α-amylase activity can be determined by measuring the amount of reducing ends produced by the enzymatic degradation of starch. The determination method is not limited thereto; for example, the α-amylase activity can also be determined by measuring the release of dye by the enzymatic degradation of dye-crosslinked starch, such as Phadebas (Soininen, K., M. Ceska, and H. Adlercreutz. "Comparison between a new chromogenic α-amylase test (Phadebas) and the Wohlgemuth amyloclastic method in urine." Scandinavian journal of clinical and laboratory investigation 30.3 (1972): 291-297.).

In the present specification, the identity of amino acid sequences or nucleotide sequences is calculated by the Lipman-Pearson method (Science, 1985, 227: 1435-1441). Specifically, the identity is calculated by analysis using a homology analysis (Search homology) program of genetic information processing software GENETYX Ver. 12 at a unit size to compare (ktup) of 2.

In the present specification, the "amino acid residues" refer to 20 amino acid residues constituting protein, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

In the present specification, amino acid positions and mutants are denoted as shown below using the officially recognized IUPAC one-letter amino acid abbreviations.

An amino acid at a predetermined position is denoted as [amino acid, position]. For example, threonine at position 226 is denoted as "T226."

"Substitution" of an amino acid is denoted as [original amino acid, position, substituted amino acid]. For example, substitution of threonine at position 226 with alanine is expressed as "T226A."

"Deletion" of an amino acid is denoted as [original amino acid, position, Δ]. For example, deletion of serine at position 181 is expressed as "S181Δ."

Mutants including multiple modifications are denoted by using the addition symbol ("+"). For example, "R170Y+G195E" represents substitution of arginine at position 170 with tyrosine and substitution of glycine at position 195 with glutamic acid, respectively.

When it is possible to introduce different modifications at one position, the different modifications are divided by the diagonal ("/"). For example, "R170Y/E" represents substitution of arginine at position 170 with tyrosine or glutamic acid.

In the present specification, the "operable linkage" between a regulatory region, such as a promoter, and a gene means that the gene and the regulatory region are linked so that the gene can be expressed under the control of the regulatory region. Procedures for the "operable linkage" between the gene and the regulatory region are well known to a person skilled in the art.

In the present specification, the "upstream" and "downstream" relating to a gene refer to the upstream and downstream in the transcription direction of the gene. For example, "a gene located downstream of a promoter" means that the gene is present on the 3' side of the promoter in the DNA sense strand, and the upstream of a gene means a region on the 5' side of the gene in the DNA sense strand.

In the present specification, the term "original" used for a function, property, or trait of a cell is used to indicate that the function, property, or trait is inherent in the cell. In contrast, the term "foreign" is used to describe a function, property, or trait that is introduced from outside the cell, rather than being inherent in the cell. For example, a "foreign" gene or polynucleotide is a gene or polynucleotide introduced into the cell from outside. The foreign gene or polynucleotide may be derived from an organism of the same species as the cell into which the foreign gene or polynucleotide is introduced, or from an organism of a different species (i.e., heterologous gene or polynucleotide).

### <Mutant>

The mutants of the present invention are α-amylase mutants A1, B1, C1, D1, and E1 derived from the following parent amylases: an α-amylase A having at least 90% sequence identity to the amino acid sequence of SEQ ID NO: 4; an α-amylase B having at least 90% sequence identity to the amino acid sequence of SEQ ID NO: 6; an α-amylase C having at least 90% sequence identity to the amino acid sequence of SEQ ID NO: 8; an α-amylase D having at least 90% sequence identity to the amino acid sequence of SEQ ID NO: 10; and an α-amylase E having at least 90% sequence identity to the amino acid sequence of SEQ ID NO: 12, respectively, wherein the mutants A1 to E1 each comprises deletion of amino acid residues at two positions selected from the group consisting of positions corresponding to positions 178 to 181 according to the numbering of SEQ ID NO: 1, and at least the following substitutions of amino acid residues at three or more positions selected from the group consisting of positions corresponding to positions 178, 181, 238, 239, 240, 126, 129, and 201 according to the numbering of SEQ ID NO: 2:
a) in A1, substitution of an amino acid residue at a position corresponding to position 178 with H, substitution of an amino acid residue at a position corresponding to position 181 with E or Q, and substitution of an amino acid residue at a position corresponding to position 239 with Q;
b) in B1, substitution of an amino acid residue at a position corresponding to position 178 with H, substitution of an amino acid residue at a position corresponding to position 181 with E or Q, and substitution of an amino acid residue at a position corresponding to position 239 with Q;
c) in C1, substitution of amino acid residues at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q;
d) in D1, substitution of amino acid residues at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q; and
e) in E1, substitution of amino acid residues at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F.

That is, the "mutants" mean polypeptides having α-amylase activity obtained by, in amino acids constituting the parent α-amylases A to E, performing a combination of: deletion of amino acid residues at two positions selected from the group consisting of positions corresponding to positions 178 to 181 according to the numbering of SEQ ID NO: 1; and at least the amino acid residue substitutions a) to e) described above at three or more positions selected from the group consisting of positions corresponding to positions 178, 181, 238, 239, 240, 126, 129, and 201 according to the numbering of SEQ ID NO: 2.

The deletion and substitution of amino acid residues at such predetermined positions are modifications intended for enhancing the stability at low pHs and/or in the presence of a chelating agent. Therefore, such mutants have enhanced stability at low pHs and/or in the presence of a chelating agent in comparison with the parent α-amylases.

The "parent α-amylases" mean standard α-amylases to be modified to bring about the mutants of the present invention, and are, in the present invention, an α-amylase A having at least 90% sequence identity to the amino acid sequence of SEQ ID NO: 4, an α-amylase B having at least 90% sequence identity to the amino acid sequence of SEQ ID NO: 6, an α-amylase C having at least 90% sequence identity to the amino acid sequence of SEQ ID NO: 8, an α-amylase D having at least 90% sequence identity to the amino acid sequence of SEQ ID NO: 10, and an α-amylase E having at least 90% sequence identity to the amino acid sequence of SEQ ID NO: 12.

Such parent amylases may be natural (wild-type) polypeptides or mutants thereof.

The α-amylase A consisting of the amino acid sequence of SEQ ID NO: 4 is α-amylase LABM of the A and B domains of *Bacillus*-derived α-amylase AAI10 and the C domain of *Alicyclobacillus-*derived α-amylase.

The α-amylase B consisting of the amino acid sequence of SEQ ID NO: 6 is Cytophaga-derived α-amylase CspAmy2.

The α-amylase C consisting of the amino acid sequence of SEQ ID NO: 8 is *Bacillus* sp. DSM12649 strain-derived α-amylase AA560.

The α-amylase D consisting of the amino acid sequence of SEQ ID NO: 10 is *Bacillus* sp. SP722 strain-derived α-amylase SP722.

The α-amylase E consisting of the amino acid sequence of SEQ ID NO: 12 is *Bacillus* sp. KSM-1378 (FERM BP-3048) strain-derived α-amylase AP1378.

Examples of the parent α-amylases include polypeptides having at least 90%, more preferably at least 95%, more preferably at least 96%, more preferably at least 97%, more preferably at least 98%, and more preferably at least 99% sequence identity to the amino acid sequence of SEQ ID NO: 4, 6, 8, 10, or 12.

In the mutants A1, B1, C1, D1, and E1 of the present invention, the modification sites (mutation positions) of amino acid residues are deletion of amino acid residues at two positions selected from the group consisting of positions corresponding to positions 178 to 181 according to the numbering of SEQ ID NO: 1, and substitution of amino acid residues at three or more positions selected from the group consisting of positions corresponding to positions 178, 181, 238, 239, 240, 126, 129, and 201 according to the numbering of SEQ ID NO: 2.

SEQ ID NO: 1 is the amino acid sequence constituting α-amylase YR288 (WO 2022/017728) registered as WP_100346362.1 in the NCBI protein sequence database, and SEQ ID NO: 2 is the amino acid sequence constituting (R178Δ+T180Δ) α-amylase in which amino acid residues corresponding to R178 and T180 in the amino acid sequence of SEQ ID NO: 1 are deleted.

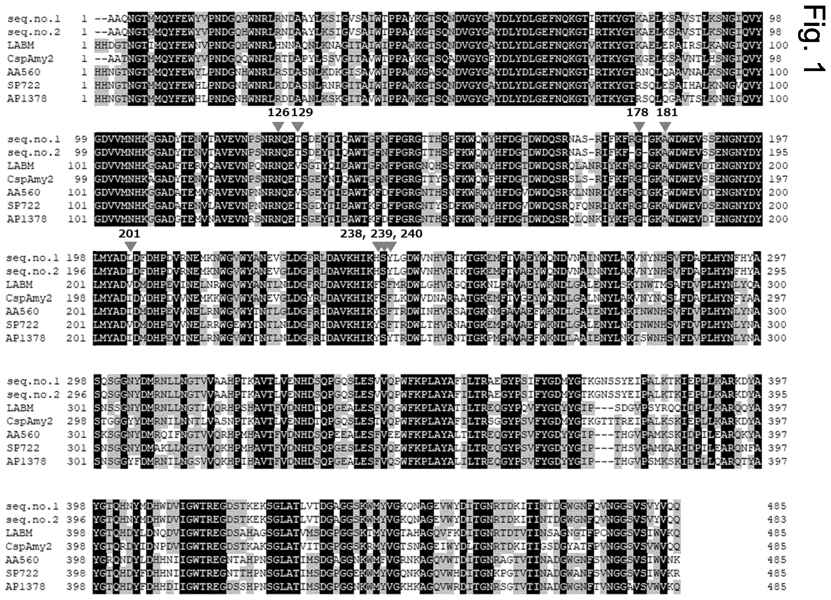

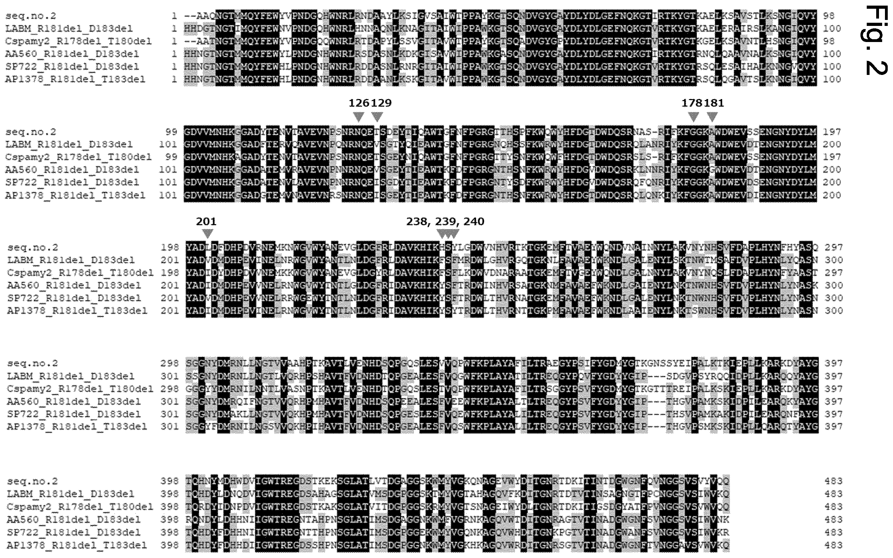

Fig. 1 shows the alignment of the amino acid sequence of SEQ ID NO: 1, the amino acid sequence of SEQ ID NO: 2, and the amino acid sequences of the parent α amylases A, B, C, D, and E described above. Further, Fig. 2 shows the alignment of the amino acid sequence of SEQ ID NO: 2 and the amino acid sequences of α-amylases in which amino acid residues at two positions corresponding to positions 178 and 180 according to the numbering of SEQ ID NO: 1 are deleted in the above parent α-amylase A, B, C, D, and E.

Regarding the parent α-amylases A to E, the following Table 1 shows the correspondence relationship between amino acid residues at positions corresponding to positions 178 to 181 according to the numbering of SEQ ID NO: 1, and amino acid residues at positions corresponding to positions 178, 181, 238, 239, 240, 126, 129, and 201 according to the numbering of SEQ ID NO: 2.

**[Table 1]**

| **Parent amylase** | **Numbering of SEQ ID NO: 1** | | | | **Numbering of SEQ ID NO: 2** | | | | | | | |
|---|---|---|---|---|---|---|---|---|---|---|---|---|
| | **178** | **179** | **180** | **181** | **178** | **181** | **238** | **239** | **240** | **126** | **129** | **201** |
| A (LABM) | R181 | G182 | D183 | G184 | G182 | A186 | F243 | S244 | F245 | N128 | V131 | V206 |
| B (CspAmy2) | R178 | G179 | T180 | G181 | G179 | A183 | F240 | S241 | F242 | N126 | T129 | 1203 |
| C (AA560) | R181 | G182 | D183 | G184 | G182 | G186 | Y243 | S244 | F245 | N128 | V131 | 1206 |
| D (SP722) | R181 | G182 | D183 | G184 | G182 | A186 | Y243 | S244 | F245 | N128 | 1131 | V206 |
| E (AP1378) | R181 | G182 | T183 | G184 | G182 | A186 | Y243 | S244 | Y245 | N128 | 1131 | 1206 |

In the present invention, the "corresponding position" on the amino acid sequence can be determined by aligning the target sequence and the reference sequence (the amino acid sequence set forth in SEQ ID NO: 2 in the present invention) so as to give maximum homology. Alignment of the amino acid sequences can be performed using known algorithms, the procedure of which is known to a person skilled in the art. For example, alignment can be performed by using the Clustal W multiple alignment program (Thompson, J.D. et al., 1994, Nucleic Acids Res. 22: 4673-4680) with default settings. Alternatively, Clustal W2 and Clustal omega, which are revised versions of Clustal W, can also be used. Clustal W, Clustal W2, and Clustal omega are available on the website of, for example, the European Bioinformatics Institute (EBI [www.ebi.ac.uk/index.html]) or the Japan DNA Data Bank operated by National Institute of Genetics (DDBJ [www.ddbj.nig.ac.jp/searches-j.html]). A position in the target sequence that is aligned to arbitrary position in the reference sequence by the above alignment is regarded as the "position corresponding" to the arbitrary position.

A person skilled in the art can further refine the alignment of the amino acid sequences obtained above to optimize them. Such optimal alignment is preferably determined in consideration of the similarity of amino acid sequences, the frequency of inserted gaps, and the like. The similarity of amino acid sequences as mentioned herein refers to, when two amino acid sequences are aligned, the ratio (%) of the number of positions where the same or similar amino acid residues are present in both sequences to the number of full-length amino acid residues. Similar amino acid residues refer to, among the 20 amino acids constituting protein, amino acid residues which have similar properties to each other in terms of polarity and charge, and which undergo so-called conservative substitution. A group consisting of such similar amino acid residues is well known to a person skilled in the art, and examples include, but are not limited to, arginine and lysine or glutamine; glutamic acid and aspartic acid or glutamine; serine and threonine or alanine; glutamine and asparagine or arginine; leucine and isoleucine; and the like.

In the mutants A1, B1, C1, D1, and E1 of the present invention, examples of the deletion of amino acid residues at two positions selected from the group consisting of positions corresponding to positions 178 to 181 according to the numbering of SEQ ID NO: 1 preferably include 178Δ+180Δ, 179Δ+180Δ, 178Δ+179Δ, 178A+181A, 179A+181A, and the like, and more preferably 178A+180A.

In the mutants A1, B1, C1, D1, and E1 of the present invention, the "substitution" of amino acid residues at three or more positions selected from the group consisting of positions corresponding to positions 178, 181, 238, 239, 240, 126, 129, and 201 according to the numbering of SEQ ID NO: 2 means that amino acids at these positions are substituted with specific amino acids, and includes at least the following embodiments a) to e) for A1, B1, C1, D1, and E1.

The number of substitution sites of amino acid residues can be 3 or more, but may be 4 or more, 5 or more, or 6 or more, from the viewpoint of stability at low pHs and/or in the presence of a chelating agent.

a) In A1, substitution at three positions including substitution of an amino acid residue at a position corresponding to position 178 with H (178H), substitution of an amino acid residue at a position corresponding to position 181 with E or Q (181E or 181Q), and substitution of an amino acid residue at a position corresponding to position 239 with Q (239Q) (178H+181E or 181Q+239Q).
b) In B1, substitution at three positions including substitution of an amino acid residue at a position corresponding to position 178 with H (178H), substitution of an amino acid residue at a position corresponding to position 181 with E or Q (181E or 181Q), and substitution of an amino acid residue at a position corresponding to position 239 with Q (239Q) (178H+181E or 181Q+239Q).
c) In C1, substitution at three or more positions selected from the group consisting of substitution of an amino acid residue at a position corresponding to position 178 with H (178H), substitution of an amino acid residue at a position corresponding to position 181 with E or Q (181E or 181Q), substitution of an amino acid residue at a position corresponding to position 238 with F (238F), and substitution of an amino acid residue at a position corresponding to position 239 with Q (239Q). For example, substitution at four positions of 178H+181E or 181Q+238F+239Q.
d) In D1, substitution at three or more positions selected from the group consisting of substitution of an amino acid residue at a position corresponding to position 178 with H (178H), substitution of an amino acid residue at a position corresponding to position 181 with E or Q (181E or 181Q), substitution of an amino acid residue at a position corresponding to position 238 with F (238F), and substitution of an amino acid residue at a position corresponding to position 239 with Q (239Q). For example, substitutions at four positions of 178H+181E or 181Q+238F+239Q.
e) In E1, substitution at three or more positions selected from the group consisting of substitution of an amino acid residue at a position corresponding to position 178 with H (178H), substitution of an amino acid residue at a position corresponding to position 181 with E or Q (181E or 181Q), substitution of an amino acid residue at a position corresponding to position 238 with F (238F), substitution of an amino acid residue at a position corresponding to position 239 with Q (239Q), and substitution of an amino acid residue at a position corresponding to position 240 with F. For example, substitution at four positions of 178H+181E or 181Q+239Q+240F and substitution at five positions of 178H+181E+238F+239Q+240F.

In a) to e) above, when the amino acid residue at a position corresponding to position 178 according to the numbering of SEQ ID NO: 2 is H, the amino acid residue at a position corresponding to position 181 is E or Q, the amino acid residue at a position corresponding to position 238 is F, the amino acid residue at a position corresponding to position 239 is Q, or the amino acid residue at a position corresponding to position 240 is F in each corresponding parent amylase A, B, C, D, or E, the amino acid residues are the same before and after substitution. Such embodiments are also included in the mutants of the present invention.

In addition to the above amino acid residue substitutions a) to e), it is also preferable to further perform, in combination, substitution of amino acid residue(s) at one or more positions selected from the group consisting of positions corresponding to positions 126, 129, and 201, from the viewpoint of enhancing stability at low pHs and/or in the presence of a chelating agent.

Examples of the substitution include substitution of an amino acid residue at a position corresponding to position 126 with Y (126Y), substitution of an amino acid residue at a position corresponding to position 129 with I (129I), and substitution of an amino acid residue at a position corresponding to position 201 with L or Y (201L/Y).

For example, in A1, B1, and C1, two substitutions 126Y+129I or one substitution 201L/Y are/is preferably further added to the above amino acid residue substitutions.

In D1, one substitution 126Y or 201L/Y is preferably further added to the above amino acid residue substitutions.

The mutants are preferably α-amylases having at least 80%, preferably at least 85%, more preferably at least 90%, preferably at least 95%, more preferably at least 96%, more preferably at least 97%, more preferably at least 98%, and more preferably at least 99% identity to the amino acid sequences of the parent amylases, from the viewpoint of enhancing cleaning performance and stability at low pHs and/or in the presence of a chelating agent.

The mutant may contain arbitrary number of conservative amino acid substitutions as long as it retains the properties as the above mutant.

### <Polynucleotide encoding the mutant of the present invention>

The mutant of the present invention can be produced by using various mutagenesis techniques known in this technical field. For example, the mutant of the present invention can be produced by mutating a polynucleotide encoding an amino acid residue to be modified in a parent α-amylase gene encoding its standard amino acid sequence (standard α-amylase gene) to a polynucleotide encoding the modified amino acid residue, and then allowing the mutated gene to express a mutant.

The polynucleotide encoding the mutant of the present invention can be in the form of single- or double-stranded DNA, RNA, or an artificial nucleic acid, or may be cDNA or chemically synthesized intron-free DNA.

In the present invention, as the means for mutating an amino acid residue of the parent α-amylase, various mutagenesis techniques known in this technical field can be used. For example, the polynucleotide encoding the mutant of the present invention can be obtained by mutating, in a polynucleotide encoding the amino acid residue of the parent α-amylase (hereinafter also referred to as the "parent gene"), a nucleotide sequence encoding an amino acid residue to be mutated to a nucleotide sequence encoding the mutated amino acid residue.

Introduction of the target mutation into the parent gene can be basically performed by various site-directed mutagenesis methods well-known to a person skilled in the art. The site-directed mutagenesis method can be performed by any method, such as an inverse PCR method or an annealing method. It is also possible to use commercially available site-directed mutagenesis kits (e.g., Stratagene's QuickChange II Site-Directed Mutagenesis Kit and QuickChange Multi Site-Directed Mutagenesis Kit, or the like).

Most commonly, site-directed mutagenesis of the parent gene can be performed by using a mutation primer containing the nucleotide mutation to be introduced. The mutation primer may be designed to be annealed to a region containing a nucleotide sequence encoding an amino acid residue to be mutated in the parent gene, and to contain a nucleotide sequence having a nucleotide sequence (codon) encoding the mutated amino acid residue in place of the nucleotide sequence (codon) encoding the amino acid residue to be mutated. The nucleotide sequences (codons) encoding the unmutated or mutated amino acid residues can be appropriately recognized and selected by a person skilled in the art based on ordinary textbooks and the like. Alternatively, site-directed mutagenesis can also be performed by using a method in which DNA fragments, obtained by amplifying the upstream and downstream sides of the mutation site separately using two complementary primers containing the nucleotide mutation to be introduced, are linked into one by SOE (splicing by overlap extension)-PCR (Gene, 1989, 77(1): pp. 61-68).

A template DNA containing the parent gene can be prepared by extracting genome DNA from a microorganism producing the α-amylase described above by a standard method, or extracting RNA and synthesizing cDNA with reverse transcription. Alternatively, a corresponding nucleotide sequence may be chemically synthesized based on the amino acid sequence of the parent α-amylase, and used as the template DNA. A DNA sequence containing a base sequence encoding the α-amylase A (LABM) consisting of the amino acid sequence of SEQ ID NO: 4 is shown in SEQ ID NO: 3, a DNA sequence containing a base sequence encoding the α-amylase B (CspAmy2) consisting of the amino acid sequence of SEQ ID NO: 6 is shown in SEQ ID NO: 5, a DNA sequence containing a base sequence encoding the α-amylase C (AA560) consisting of the amino acid sequence of SEQ ID NO: 8 is shown in SEQ ID NO: 7, a DNA sequence containing a base sequence encoding the α-amylase D (SP722) consisting of the amino acid sequence of SEQ ID NO: 10 is shown in SEQ ID NO: 9, and a DNA sequence containing a base sequence encoding the α-amylase E (AP1378) consisting of the amino acid sequence of SEQ ID NO: 12 is shown in SEQ ID NO: 11.

A mutation primer can be produced by well-known oligonucleotide synthesis methods, such as the phosphoramidite method (Nucleic Acids R4esearch, 1989, 17: 7059-7071). Such primer synthesis can also be performed using, for example, a commercially available oligonucleotide synthesizer (e.g., ABI). Using a primer set containing the mutation primer, site-directed mutagenesis as described above can be carried out using the parent gene as the template DNA, thereby obtaining a polynucleotide encoding the mutant of the present invention having the target mutation.

The polynucleotide encoding the mutant of the present invention can contain single- or double-stranded DNA, cDNA, RNA, or other artificial nucleic acids. The DNA, cDNA, and RNA may be chemically synthesized. The polynucleotide may contain nucleotide sequences of untranslated regions (UTRs) in addition to open reading frames (ORFs). The codon of the polynucleotide may be optimized depending on the species of the transformant used for the production of the mutant polynucleotide of the present invention. Information on codons used by various organisms is available from the Codon Usage Database ([www.kazusa.or.jp/codon/]).

### <Vector or DNA fragment>

The obtained polynucleotide encoding the mutant of the present invention can be incorporated into a vector. The type of vector to contain the polynucleotide is not particularly limited, and any vector, such as a plasmid, phage, phagemid, cosmid, virus, YAC vector, or shuttle vector, may be used. The vector is not limited, but is preferably a vector which can be amplified in bacteria, preferably *Bacillus* bacteria (e.g., *Bacillus subtilis* or mutant strains thereof), and more preferably an expression vector which can induce the expression of transgenes in *Bacillus* bacteria. Among these, shuttle vectors, which are vectors replicable in *Bacillus* bacteria and any other organisms, can be preferably used in the recombinant production of the mutant of the present invention. Examples of preferred vectors include, but are not limited to, pHA3040SP64, pHSP64R, or pASP64 (JP-B-3492935), pHY300PLK (an expression vector which can transform both *Escherichia coli* and *Bacillus subtilis;* Jpn J Genet, 1985, 60: 235-243), pAC3 (Nucleic Acids Res, 1988, 16: 8732), and other shuttle vectors; pUB110 (J Bacteriol, 1978, 134: 318-329), pTA10607 (Plasmid, 1987, 18: 8-15), and other plasmid vectors which can be used in the transformation of *Bacillus* bacteria; and the like. Other usable examples include *Escherichia* coli-derived plasmid vectors (e.g., pET22b(+), pBR322, pBR325, pUC57, pUC118, pUC119, pUC18, pUC19, and pBluescript, and the like).

The above vector may contain a DNA replication initiation region or a DNA region containing a replication origin. Alternatively, in the above vector, a regulatory sequence, such as a promoter region for initiating the transcription of the gene, a terminator region, or a secretion signal region for secreting the expressed protein outside the cell, may be operably linked to the upstream of the polynucleotide encoding the mutant of the present invention (i.e., mutant gene). The phrase that a gene and a regulatory sequence are "operably linked" means that the gene and the regulatory region are arranged so that the gene can be expressed under the control of the regulatory region.

The type of regulatory sequence, such as a promoter region, a terminator, or a secretion signal region mentioned above, is not particularly limited, and generally used promoters and secretion signal sequences can be appropriately selected depending on the host for introduction. Examples of preferred regulatory sequences that can be incorporated into the vector include the promoter, secretion signal sequence, and the like of the cellulase gene of *Bacllus* sp. KSM-S237 strain.

Alternatively, a marker gene (e.g., a gene resistant to drugs, such as ampicillin, neomycin, kanamycin, and chloramphenicol) for selecting the host into which the vector of the present invention is appropriately introduced may be further incorporated into the vector. Alternatively, when an auxotroph is used as the host, a gene encoding the desired nutritional synthetic enzyme may be incorporated as a marker gene into the vector. Alternatively, when a selective culture medium in which a specific metabolism is required for growth, is used, a gene associated with the metabolism may be incorporated as a marker gene into the vector. Examples of such metabolism-related gene include acetamidase genes for utilizing acetamide as a nitrogen source.

The polynucleotide encoding the mutant of the present invention, a regulatory sequence, and a marker gene can be linked by a method known in the art, such as SOE (splicing by overlap extension)-PCR (Gene, 1989, 77: 61-68). Procedures for introducing the linked fragment into the vector are well known in the art.

### <Transformed cell>

The transformed cell of the present invention can be obtained by introducing a vector containing the polynucleotide encoding the mutant of the present invention into a host, or by introducing a DNA fragment containing the polynucleotide encoding the mutant of the present invention into the genome of the host.

Examples of the host cells include microorganisms, such as bacteria and filamentous fungi. Examples of bacteria include *Escherichia coli* and bacteria belonging to the genera *Staphylococcus, Enterococcus, Listeria,* and *Bacillus.* Preferred among these are *Escherichia coli* and *Bacillus* bacteria (e.g., *Bacillus subtilis* Marburg No. *168 (Bacillus subtilis* 168 strain) or mutant strains thereof). Examples of *Bacillus subtilis* mutant strains include the nine-protease-deficient strain KA8AX described in J. Biosci. Bioeng., 2007, 104(2): 135-143, and the eight-protease-deficient strain with improved protein folding efficiency, D8PA strain, described in Biotechnol. Lett., 2011, 33(9): 1847-1852. Examples of filamentous fungi include *Trichoderma, Aspergillus, Rizhopus,* and the like.

Methods commonly used in the art, such as the protoplast method and the electroporation method, can be used to introduce the vector into the host. Strains with appropriate introduction are selected using marker gene expression, auxotrophy, and the like as indices, whereby the target transformant into which the vector is introduced can be obtained.

Alternatively, a fragment obtained by linking the polynucleotide encoding the mutant of the present invention, a regulatory sequence, and a marker gene can also be introduced directly into the genome of the host. For example, a DNA fragment in which sequences complementary to the genome of the host are added to both ends of the linked fragment is constructed by the SOE-PCR method or the like, and this DNA fragment is then introduced into the host to induce homologous recombination between the host genome and the DNA fragment, whereby the polynucleotide encoding the mutant of the present invention is introduced into the genome of the host.

When the thus-obtained transformant into which the polynucleotide encoding the mutant of the present invention, or a vector containing the polynucleotide is introduced, is cultured in a suitable culture medium, the gene encoding the protein on the vector is expressed to produce the mutant of the present invention. The culture medium used for culturing the transformant can be appropriately selected by a person skilled in the art depending on the type of microorganism of the transformant.

Alternatively, the mutant of the present invention may be expressed from the polynucleotide encoding the mutant of the present invention or a transcript thereof using a cell-free translation system. The "cell-free translation system" is such that reagents, such as amino acids, necessary for the protein translation are added to a suspension obtained by mechanically destroying a cell, which serves as the host, to construct an in vitro transcription-translation system or an in vitro translation system.

The mutant of the present invention produced in the above culture or cell-free translation system can be isolated or purified by using general methods used for protein purification, such as centrifugation, ammonium sulfate precipitation, gel chromatography, ion-exchange chromatography, and affinity chromatography, singly or in a suitable combination. In this case, when the gene encoding the α-amylase mutant of the present invention is operably linked to a secretion signal sequence on the vector in the transformant, the produced protein is secreted extracellularly, and can be thus more easily collected from the culture. The protein collected from the culture may be further purified by known means.

The thus-obtained mutant of the present invention has enhanced stability at low pHs and/or in the presence of a chelating agent in comparison with the parent α-amylase.

The "enhanced stability at low pHs and/or in the presence of a chelating agent" means an enhanced ability to maintain α-amylase activity in a low-pH environment and/or in the presence of a chelating agent, in comparison with the parent α-amylase.

The low pH refers to weak acidity, i.e., a pH from 4 to 7, and preferably a pH from 4.5 to 6.5, and the stability in a low-pH environment means that α-amylase activity is maintained in a cleaning composition with a pH from about 4 to about 7, and that α-amylase activity is maintained in cleaning water with a pH from about 4 to about 7 prepared by dissolving or diluting the cleaning composition in water.

Examples of chelating agents include chelating agents which can be incorporated into cleaning compositions, such as amino carboxylic acid-based chelating agents, phosphonic acid-based chelating agents, hydroxycarboxylic acid-based chelating agents, and polyhydric carboxylic acid-based chelating agents, described later.

The stability at low pHs and/or in the presence of a chelating agent can be evaluated by using a method well known in the art. For example, an enzyme solution is added to a cleaning composition with a pH from 4 to 7 containing a chelating agent, the α-amylase activity is measured before and after treatment for a predetermined time, the activity value of the sample before treatment is regarded as the initial activity, and the ratio of the activity value after treatment to the initial activity is regarded as the remaining activity (%).

The mutant of the present invention is useful as an enzyme to be incorporated into various cleaning compositions, and particularly useful as an enzyme to be incorporated into acidic and chelating agent-incorporated cleaning compositions suitable for low-temperature cleaning at 40°C or lower.

The amount of the mutant of the present invention incorporated into the cleaning composition is not particularly limited as long as the protein can exhibit activity. For example, the amount of the protein per kg of the cleaning composition is preferably 1 mg or more, more preferably 10 mg or more, and even more preferably 50 mg or more, as well as preferably 5,000 mg or less, more preferably 1,000 mg or less, and even more preferably 500 mg or less. The amount of the protein is also preferably from 1 to 5,000 mg, more preferably from 10 to 1,000 mg, and even more preferably from 50 to 500 mg.

In the cleaning composition, various enzymes other than the mutant of the present invention can be used in combination. Examples include hydrolases, oxidases, reductases, transferases, lyases, isomerases, ligases, synthetases, and the like. Preferred among these are amylases different from the protein of the present invention, proteases, cellulases, keratinases, esterases, cutinases, lipases, pullulanases, pectinases, mannanases, glucosidases, glucanases, cholesterol oxidases, peroxidases, laccases, and the like; and particularly preferred are proteases, cellulases, amylases, and lipases.

Examples of proteases include commercially available Alcalase, Esperase, Everlase, Savinase, Kannase, and Progress Uno (registered trademarks; Novozymes A/S), PREFERENZ, EFFECTENZ, and EXCELLENZ (registered trademarks; DuPont), Lavergy (registered trademark; BASF), KAP (Kao Corporation), and the like.

Examples of cellulases include Celluclean and Carezyme (registered trademarks; Novozymes A/S); KAC, the alkaline cellulase produced by *Bacillus sp.* KSM-S237 strain described in JP-A-10-313859, and the mutant alkaline cellulase described in JP-A-2003-313592 (Kao Corporation); and the like.

Examples of amylases include Termamyl, Duramyl, Stainzyme, Stainzyme Plus, and Amplify Prime (registered trademarks; Novozymes A/S), PREFERENZ and EFFECTENZ (registered trademarks; DuPont), KAM (Kao Corporation), and the like.

Examples of lipases include Lipolase and Lipex (registered trademarks; Novozymes A/S), and the like.

Known cleaning agent components can be incorporated into the cleaning composition, and examples of such known cleaning agent components include the following.

### (1) Surfactant

The surfactant may be contained in an amount of from 0.5 to 60 mass% in the cleaning composition, and preferably from 10 to 45 mass% particularly in a powder cleaning composition, and from 20 to 90 mass% in a liquid cleaning composition. When the cleaning composition of the present invention is a clothing cleaning agent for laundry or a cleaning agent for an automatic dishwasher, the surfactant is generally contained in an amount of from 1 to 10 mass%, and preferably from 1 to 5 mass%.

Examples of the surfactant used in the cleaning composition include one or a combination of anionic surfactants, non-ionic surfactants, amphoteric surfactants, and cationic surfactants; and anionic surfactants and non-ionic surfactants are preferred.

Examples of preferred anionic surfactants include sulfate ester salts of alcohols having from 10 to 18 carbon atoms, sulfate ester salts of alkoxylated alcohols having from 8 to 20 carbon atoms, alkylbenzene sulfonate, paraffin sulfonate, α-olefin sulfonate, internal olefin sulfonate, α-sulfo fatty acid salts, α-sulfo fatty acid alkyl ester salts, and fatty acid salts. In the present invention, particularly preferred is one or more anionic surfactants selected from the group consisting of linear alkylbenzene sulfonate with an alkyl chain having from 10 to 14 carbon atoms, more preferably from 12 to 14 carbon atoms, and internal olefin sulfone with an alkylene chain having from 12 to 20 carbon atoms, more preferably from 16 to 18 carbon atoms. Alkali metal salts and amines are preferable as counterions, and sodium and/or potassium, monoethanolamine, and diethanolamine are particularly preferred. For internal olefin sulfonic acid, reference can be made to, for example, WO 2017/098637.

Preferred non-ionic surfactants are polyoxyalkylene alkyl (from 8 to 20 carbon atoms) ether, alkyl polyglycoside, polyoxyalkylene alkyl (from 8 to 20 carbon atoms) phenyl ether, polyoxyalkylene sorbitan fatty acid (from 8 to 22 carbon atoms) ester, polyoxyalkylene glycol fatty acid (from 8 to 22 carbon atoms) ester, and polyoxyethylene polyoxypropylene block polymers. In particular, preferred non-ionic surfactants are polyoxyalkylene alkyl ethers obtained by adding 4 to 20 moles of alkylene oxides, such as ethylene oxide and propylene oxide, to alcohols having from 10 to 18 carbon atoms [an HLB value (calculated by the Griffin method) of from 10.5 to 15.0, and preferably from 11.0 to 14.5].

### (2) Divalent metal ion scavenger

A divalent metal ion scavenger may be contained in an amount of from 0.01 to 50 mass%, and preferably from 5 to 40 mass%. Examples of the divalent metal ion scavenger used in the cleaning composition of the present invention include condensed phosphates, such as tripolyphosphates, pyrophosphates, and orthophosphates; aluminosilicates, such as zeolites; synthetic layered crystalline silicates, nitrilotriacetates, ethylenediaminetetraacetates, citrates, isocitrates, polyacetal carboxylates, and the like. Among these, crystalline aluminosilicates (synthetic zeolites) are particularly preferred. Among A-, X-, and P-type zeolites, A-type zeolites are particularly preferred. As synthetic zeolites, those having an average primary particle size of from 0.1 to 10 µm, particularly from 0.1 to 5 µm, are preferably used.

### (3) Alkali agent

An alkali agent may be contained an amount of from 0.01 to 80 mass%, preferably from 1 to 40 mass%. In the case of powder cleaning agents, examples of alkali agents include alkali metal carbonates, such as sodium carbonate, collectively called dense ash or light ash; and amorphous alkali metal silicates, such as JIS Nos. 1, 2, and 3. These inorganic alkali agents are effective in the formation of the particle skeleton during drying of cleaning agent, and relatively hard cleaning agents having excellent flowability can be obtained. Examples of other alkalis include sodium sesquicarbonate, sodium hydrogencarbonate, and the like. In addition, phosphates, such as tripolyphosphates, also have the action as alkali agents. As alkali agents used in liquid cleaning agents, sodium hydroxide and mono-, di-, or triethanolamine can be used, in addition to the alkali agents mentioned above, and they can also be used as counterions of activators.

### (4) Anti-redeposition agent

The anti-redeposition agent may be contained in an amount of from 0.001 to 10 mass%, preferably from 1 to 5 mass%. Examples of the anti-redeposition agent used in the cleaning composition of the present invention include polyethylene glycol, carboxylic acid-based polymers, polyvinyl alcohol, polyvinylpyrrolidone, and the like. Among these, carboxylic acid-based polymers have the function of scavenging metal ions and the ability to disperse solid particle stains from the clothing into the laundry bath, as well as the anti-redeposition ability. Carboxylic acid-based polymers are homopolymers or copolymers of acrylic acid, methacrylic acid, itaconic acid, or the like. Preferred copolymers are copolymers of the above monomers and maleic acid, and those with a molecular weight of from several thousands to a hundred thousand are preferred. In addition to the carboxylic acid-based polymers mentioned above, polymers such as polyglycidylates, cellulose derivatives such as carboxymethyl cellulose, and amino carboxylic acid-based polymers such as polyaspartic acid are also preferred because they have metal ion scavenging, dispersion, and anti-redeposition ability.

### (5) Bleaching agent

A bleaching agent, such as hydrogen peroxide or percarbonate, may be preferably contained in an amount of from 1 to 10 mass%. When the bleaching agent is used, tetraacetylethylenediamine (TAED) or the bleach activator described in JP-A-6-316700 can be contained in an amount of from 0.01 to 10 mass%.

### (6) Fluorescent agent

Examples of the fluorescent agent used in the cleaning composition include biphenyl-type fluorescent agents (e.g., Tinopal CBS-X and the like) and stilbene-type fluorescent agents (e.g., a DM-type fluorescent dye and the like). The fluorescent agent may be preferably contained in an amount of from 0.001 to 2 mass%.

### (7) Chelating agent

A chelating agent may be contained in order to, for example, facilitate the cleaning of stains and reduce the water hardness during cleaning.

Examples of such chelating agents include amino carboxylic acid-based chelating agents, phosphonic acid-based chelating agents, hydroxycarboxylic acid-based chelating agents, polyhydric carboxylic acid-based chelating agents, and the like.

Examples of amino carboxylic acid-based chelating agents include ethylenediaminetetraacetic acid (EDTA), nitrilotriacetic acid (NTA), hydroxyethylethylenediaminetriacetic acid (HEDTA), diethylenetriaminepentaacetic acid (DTPA), methylglycinediacetic acid (MGDA), triethylenetetraminehexaacetic acid (TTHA), glutamic acid diacetic acid (GLDA), hydroxyethyliminodiacetic acid (HIDA), dihydroxyethylglycine (DHEG), aspartic acid diacetic acid (ASDA), ethylenediaminesuccinic acid (EDDS), and salts thereof.

Examples of phosphonic acid-based chelating agents include hydroxyethylidene diphosphonic acid (HEDP), nitrilotrismethylenephosphonic acid (NTMP), phosphonobutanetricarboxylic acid (PBTC), ethylenediaminetetramethylenephosphonic acid (EDTMP), and salts thereof.

Examples of hydroxycarboxylic acid-based chelating agents include citric acid, malic acid, tartaric acid, gluconic acid, lactic acid, and salts thereof.

Examples of polyhydric carboxylic acid-based chelating agents include succinic acid, oxalic acid, glutaric acid, adipic acid, fumaric acid, malonic acid, and salts thereof.

### (8) Buffer

A buffer may be contained in order to create a buffer system capable of maintaining the low pH of the cleaning liquid. Examples of such buffers include mixtures of organic acids and salts thereof, such as mixtures of polycarboxylic acids and salts thereof, preferably a mixture of citric acid and citrate.

### (9) Other components

The cleaning composition may contain builders, softeners, reducing agents (e.g., sulfite), foam inhibitors (e.g., silicone), fragrances, antibacterial and antifungal agents (e.g., Proxel [trade name] and benzoic acid), and other additives known in the field of clothing cleaning agents.

The cleaning composition can be produced in accordance with a standard method by combining the protein of the present invention obtained by the above method and the known cleaning components mentioned above. The form of the cleaning agent can be selected depending on the application. For example, the cleaning agent can be in the form of liquid, powder, granules, paste, solids, or the like.

The thus-obtained cleaning composition can be used as a clothing cleaning agent, a dishwashing cleaning agent, a bleaching agent, a cleaning agent for hard surface cleaning, a drain cleaning agent, a denture cleaning agent, a disinfecting cleaning agent for medical instruments, or the like; preferably a clothing cleaning agent and a dishwashing cleaning agent; and more preferably a clothing cleaning agent for laundry (laundry cleaning agent), a dishwashing cleaning agent for hand washing, and a cleaning agent for an automatic dishwasher.

The cleaning composition is also suitable for use at low temperatures (e.g., 40°C or lower, 35°C or lower, 30°C or lower, or 25°C or lower, and at 5°C or higher, 10°C or higher, or 15°C or higher) .

Regarding the embodiments described above, the present invention further discloses the following aspects.
<1> α-Amylase mutants A1, B1, C1, D1, and E1 derived from the following parent amylases: an α-amylase A having at least 90% sequence identity to the amino acid sequence of SEQ ID NO: 4; an α-amylase B having at least 90% sequence identity to the amino acid sequence of SEQ ID NO: 6; an α-amylase C having at least 90% sequence identity to the amino acid sequence of SEQ ID NO: 8; an α-amylase D having at least 90% sequence identity to the amino acid sequence of SEQ ID NO: 10; and an α-amylase E having at least 90% sequence identity to the amino acid sequence of SEQ ID NO: 12, respectively, wherein the mutants A1 to E1 each comprises deletion of amino acid residues at two positions selected from the group consisting of positions corresponding to positions 178 to 181 according to the numbering of SEQ ID NO: 1, and substitution of amino acid residues at three or more positions selected from the group consisting of positions corresponding to positions 178, 181, 238, 239, 240, 126, 129, and 201 according to the numbering of SEQ ID NO: 2, the substitution comprising at least the following a) to e) :
   a) in A1, substitution of an amino acid residue at a position corresponding to position 178 with H, substitution of an amino acid residue at a position corresponding to position 181 with E or Q, and substitution of an amino acid residue at a position corresponding to position 239 with Q;
   b) in B1, substitution of an amino acid residue at a position corresponding to position 178 with H, substitution of an amino acid residue at a position corresponding to position 181 with E or Q, and substitution of an amino acid residue at a position corresponding to position 239 with Q;
   c) in C1, substitution at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q;
   d) in D1, substitution at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q; and
   e) in E1, substitution three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F.
<2> The α-amylase mutant according to <1>, wherein
   the substitution in C1 is substitution at four positions including substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q according to the numbering of SEQ ID NO: 2;
   the substitution in D1 is substitution at four positions including substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q according to the numbering of SEQ ID NO: 2; and
   the substitution in E1 is substitution at four or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F according to the numbering of SEQ ID NO: 2.
<3> The α-amylase mutant according to <2>, wherein the substitution in E1 is substitution at five positions including substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F according to the numbering of SEQ ID NO: 2.
<4> The α-amylase mutant according to any one of <1> to <3>, wherein the mutants further comprises, in a) to e) above, substitution of amino acid residue(s) at one or more positions selected from the group consisting of positions corresponding to positions 126, 129, and 201 according to the numbering of SEQ ID NO: 2.
<5> The α-amylase mutant according to <4>, wherein the amino acid residues after substitution at positions corresponding to positions 126, 129, and 201 in the α-amylase are 126Y, 129I, and 201L/Y, respectively.
<6> A polynucleotide encoding the mutant according to any one of <1> to <5>.
<7> A vector or DNA fragment comprising the polynucleotide according to <6>.
<8> A transformed cell comprising the vector or DNA fragment according to <7>.
<9> The transformed cell according to <8>, wherein the transformed cell is a microorganism.
<10> A cleaning composition comprising the mutant according to any one of <1> to <5>.
<11> The cleaning composition according to <10>, wherein the cleaning composition is a clothing cleaning agent or a dishwashing cleaning agent.
<12> The cleaning composition according to <11>, wherein the cleaning composition is a powder or a liquid.
<13> The cleaning composition according to <11> or <12>, wherein the cleaning composition is weakly acidic, preferably has a pH from 4 to 7.
<14> The cleaning composition according to any one of <11> to <13>, wherein the cleaning composition comprises a chelating agent.
<15> The cleansing composition according to <14>, wherein the chelating agent is an amino carboxylic acid-based chelating agent, a phosphonic acid-based chelating agent, a hydroxycarboxylic acid-based chelating agent, or a polyhydric carboxylic acid-based chelating agent.

### Examples

### (1) Construction of template α-amylase expression plasmids

Artificially synthesized LABM gene (SEQ ID NO: 3) was used as a template, and PCR was performed by using primer pair LABM_fw/LABM_rv (SEQ ID NOs: 15 and 16) and PrimeSTAR Max Premix (Takara Bio Inc.). Plasmid pHY-S237 described in Example 7 of WO 2006/068148 was used as a template, and PCR was similarly performed by using primer pair S237t_fw/S237s_rv (SEQ ID NOs: 13 and 14). Using each of the PCR products, the In-Fusion reaction was performed in accordance with the protocol of the In-Fusion, HD Cloning kit (Clontech). Using the In-Fusion reaction solution, *Bacillus subtilis* was transformed to construct plasmid pHY-LABM. In addition, plasmids pHY-Cspamy2, pHY-AA560, pHY-SP722, and pHY-AP1378 described in Japanese Patent Application No. 2021-112712 were used for the expression of Cspamy2, AA560, SP722, and AP1378, respectively.

Further, using each of these five plasmids as a template, positions corresponding to positions 178 and 180 according to the numbering of SEQ ID NO: 1 were deleted. Specifically, pHY-LABM was used as a template, and PCR was performed by using primer pair LABM_181-183del_fw/LABM_181-183del_rv (SEQ ID NOs: 17 and 18) and PrimeSTAR Max Premix (Takara Bio Inc.). Thereafter, in the same manner as described above, the In-Fusion reaction was performed, and *Bacillus subtilis* was transformed to construct plasmid pHY-LABM R181Δ D183Δ. Similarly, for Cspamy2, AA560, SP722, and AP1378, using each of pHY-Cspamy2, pHY-AA560, pHY-SP722, and pHY-AP1378 as a template, PCR and the In-Fusion reaction were performed by using primer pairs Cspamy2_178-180del_fw/Cspamy2_178-180del_rv (SEQ ID NOs: 19 and 20), AA560_181-183del_fw/AA560_181-183del_rv (SEQ ID NOs: 21 and 22), SP722_181-183del_fw/SP722_181-183del_rv (SEQ ID NOs: 23 and 24), and AP1378_181-183del_fw/AP1378_181-183del_rv (SEQ ID NOs: 25 and 26). Using the In-Fusion reaction solution, *Bacillus subtilis* was transformed to construct plasmids pHY-Cspamy2 R178Δ T180Δ, pHY-AA560 R181Δ D183Δ, pHY-SP722 R181Δ D183Δ, and pHY-AP1378 R181Δ T183Δ.

### (2) Construction of α-amylase mutant expression plasmids

Using pHY-LABM R181Δ D183Δ, pHY-Cspamy2 R178Δ T180Δ, pHY-AA560 R181Δ D183Δ, pHY-SP722 R181Δ D183Δ, or pHY-AP1378 R181Δ T183Δ described above as a template, α-amylase mutants were constructed in the manner shown below. PCR was performed by using a forward primer having 15 bases of a sequence complementary to a reverse primer at the 5'-terminal and containing a mutant sequence, and the reverse primer having a base just before the mutant sequence at the 5'-terminal as a mutagenesis primer pair. When multiple fragments were linked, using each of the PCR products, the In-Fusion reaction was performed in accordance with the protocol of the In-Fusion, HD Cloning kit (Clontech). Using the PCR product or In-Fusion reaction solution, *Bacillus subtilis* was transformed by the protoplast method to obtain a transformant retaining the target α-amylase mutant expression plasmid.

Fig. 1 shows the alignment of SEQ ID NO: 1, SEQ ID NO: 2, and five parent α-amylases created by using GENETYX ver.12, and Fig. 2 shows the alignment of SEQ ID NO: 2 and the above α-amylases with a deletion of two amino acids. Using the numbering of SEQ ID NO: 2, residues corresponding to positions 126, 129, 178, 181, 201, 238, 239, and 240 were indicated by red triangles, and the amino acid residue numbers in SEQ ID NO: 2 were shown above the red triangles. The α-amylase mutants shown in Table 2-1 and Table 2-2 containing substitutions at positions selected from these residues were constructed by the method described above.

**[Table 2-1]**

| Mutant no. | Parent enzyme | Mutation (numbering according to each parent enzyme sequence) | Residue number (numbering according to SEQ ID NO: 1) | | Residue number (numbering according to SEQ ID NO: 2) | | | | | | | |
|---|---|---|---|---|---|---|---|---|---|---|---|---|
| | | | 178 | 180 | 178 | 181 | 238 | 239 | 240 | 126 | 129 | 201 |
| no. 01 | LABM | R181Δ+G182H+D183Δ+A186E+S244Q | Δ | Δ | H | E | F | Q | F | N | V | V |
| no. 02 | CspAmy2 | R178Δ+G179H+T180Δ+A183E+S241Q | Δ | Δ | H | E | F | Q | F | N | T | I |
| no. 03 | AA560 | R181Δ+G182H+0183Δ+G186E+Y243F+S244Q | Δ | Δ | H | E | F | Q | F | N | V | I |
| no. 04 | SP722 | R181Δ+G182H+D183Δ+A186E+Y243F+S244Q | Δ | Δ | H | E | F | Q | F | N | I | V |
| no. 05 | AP1378 | R181Δ+G182H+T183Δ+A186E+Y243F+S244Q+Y245F | Δ | Δ | H | E | F | Q | F | N | I | I |
| no. 06 | LABM | N128Y+V131I+R181Δ+G182H+D183Δ+A186E+S244Q | Δ | Δ | H | E | F | Q | F | Y | I | V |
| no. 07 | CspAmy2 | N126Y+T129I+R178A+G179H+T180Δ+A183E+S241Q | Δ | Δ | H | E | F | Q | F | Y | I | I |
| no. 08 | AA560 | N128Y+V131I+R181Δ+G182H+D183Δ+G186E+Y243F +S244Q | Δ | Δ | H | E | F | Q | F | Y | I | I |
| no. 09 | SP722 | N128Y+R181Δ+G182H+D183Δ+A186E+Y243F+S244 Q | Δ | Δ | H | E | F | Q | F | Y | I | V |
| no. 10 | AP1378 | N128Y+R181Δ+G182H+T183Δ+A186E+Y243F+S244Q +Y245F | Δ | Δ | H | E | F | Q | F | Y | I | I |
| no. 11 | LABM | R181Δ+G182H+D183Δ+A186E+V206L+S244Q | Δ | Δ | H | E | F | Q | F | N | V | L |
| no. 12 | CspAmy2 | R178Δ+G179H+T180Δ+A183E+1203L+S241Q | Δ | Δ | H | E | F | Q | F | N | T | L |
| no. 13 | AA560 | R181Δ+G182H+D183Δ+G186E+1206L+Y243F+S244Q | Δ | Δ | H | E | F | Q | F | N | V | L |
| no. 14 | SP722 | R181Δ+G182H+D183Δ+A186E+V206L+Y243F+S244Q | Δ | Δ | H | E | F | Q | F | N | I | L |
| no. 15 | AP1378 | R181Δ+G182H+T183Δ+A186E+1206L+Y243F+S244Q+ Y245F | Δ | Δ | H | E | F | Q | F | N | I | L |
| no. 16 | LABM | R181Δ+G182H+D183Δ+A186E+V206Y+S244Q | Δ | Δ | H | E | F | Q | F | N | V | Y |
| no. 17 | CspAmy2 | R178Δ+G179H+T180Δ+A183E+1203Y+S241Q | Δ | Δ | H | E | F | Q | F | N | T | Y |
| no. 18 | AA560 | R181Δ+G182H+D183Δ+G186E+I206Y+Y243F+S244Q | Δ | Δ | H | E | F | Q | F | N | V | Y |
| no. 19 | SP722 | R181Δ+G182H+D183Δ+A186E+V206Y+Y243F+S244Q | Δ | Δ | H | E | F | Q | F | N | I | Y |
| no. 20 | AP1378 | R181Δ+G182H+T183Δ+A186E+I206Y+Y243F+S244Q +Y245F | Δ | Δ | H | E | F | Q | F | N | I | Y |

**[Table 2-2]**

| Mutant no. | Parent enzyme | Mutation (numbering according to each parent enzyme sequence) | Residue number (numbering according to SEQ ID NO: 1) | | Residue number (numbering according to SEQ ID NO: 2) | | | | | | | |
|---|---|---|---|---|---|---|---|---|---|---|---|---|
| | | | 178 | 180 | 178 | 181 | 238 | 239 | 240 | 126 | 129 | 201 |
| no. 21 | LABM | R181Δ+G182H+D183Δ+A186Q+S244Q | Δ | Δ | H | Q | F | Q | F | N | V | V |
| no. 22 | CspAmy2 | R178Δ+G179H+T180Δ+A183Q+S241Q | Δ | Δ | H | Q | F | Q | F | N | T | I |
| no. 23 | AA560 | R181Δ+G182H+D183Δ+G186Q+Y243F+S244Q | Δ | Δ | H | Q | F | Q | F | N | V | I |
| no. 24 | SP722 | R181Δ+G182H+D183Δ+A186Q+Y243F+S244Q | Δ | Δ | H | Q | F | Q | F | N | I | V |
| no. 25 | AP1378 | R181Δ+G182H+T183Δ+A186Q+Y243F+S244Q+Y245F | Δ | Δ | H | Q | F | Q | F | N | I | I |
| no. 26 | LABM | N128Y+V131I+R181Δ+G182H+D183Δ+A186Q+S244Q | Δ | Δ | H | Q | F | Q | F | Y | I | V |
| no. 27 | CspAmy2 | N126Y+T1291+R178Δ+G179H+T180Δ+A183Q+S241Q | Δ | Δ | H | Q | F | Q | F | Y | I | I |
| no. 28 | AA560 | N128Y+V131I+R181Δ+G182H+D183Δ+G186Q+Y243F+S244Q | Δ | Δ | H | Q | F | Q | F | Y | I | I |
| no. 29 | SP722 | N128Y+R181Δ+G182H+D183Δ+A186Q+Y243F+S244Q | Δ | Δ | H | Q | F | Q | F | Y | I | V |
| no. 30 | AP1378 | N128Y+R181Δ+G182H+T183Δ+A186Q+Y243F+S244Q+Y245F | Δ | Δ | H | Q | F | Q | F | Y | I | I |
| no. 31 | LABM | R181Δ+G182H+D183Δ+A186Q+V206L+S244Q | Δ | Δ | H | Q | F | Q | F | N | V | L |
| no. 32 | CspAmy2 | R178Δ+G179H+T180Δ+A183Q+I203L+S241Q | Δ | Δ | H | Q | F | Q | F | N | T | L |
| no. 33 | AA560 | R181Δ+G182H+D183Δ+G186Q+I206L+Y243F+S244Q | Δ | Δ | H | Q | F | Q | F | N | V | L |
| no. 34 | SP722 | R181Δ+G182H+D183Δ+A186Q+V206L+Y243F+S244Q | Δ | Δ | H | Q | F | Q | F | N | I | L |
| no. 35 | AP1378 | R181Δ+G182H+T183A+A186Q+I206L+Y243F+S244Q+Y245F | Δ | Δ | H | Q | F | Q | F | N | I | L |
| no. 36 | LABM | R181Δ+G182H+D183Δ+A186Q+V206Y+S244Q | Δ | Δ | H | Q | F | Q | F | N | V | Y |
| no. 37 | CspAmy2 | R178Δ+G179H+T180+A183Q+I203Y+S241Q | Δ | Δ | H | Q | F | Q | F | N | T | Y |
| no. 38 | AA560 | R181Δ+G182H+D183Δ+G186Q+I206Y+Y243F+S244Q | Δ | Δ | H | Q | F | Q | F | N | V | Y |
| no. 39 | SP722 | R181Δ+G182H+D183Δ+A186Q+V206Y+Y243F+S244Q | Δ | Δ | H | Q | F | Q | F | N | I | Y |
| no.40 | AP1378 | R181Δ+G182H+T183Δ+A186Q+I206Y+Y243F+S244Q+Y245F | Δ | Δ | H | Q | F | Q | F | N | I | Y |

### (3) Enzyme production culture

The recombinant *Bacillus subtilis* colonies obtained in (1) were inoculated in a 96-deep-well plate into which 500 µL of LB culture medium supplemented with 10 ppm tetracycline was dispensed, and then cultured at 32°C at 1,500 rpm overnight. Next day, 20 µL of the culture was inoculated in a 96-deep-well plate into which 500 µL of 2xL-maltose culture medium (2% tryptone, 1% yeast extract, 1% NaCl, 7.5% maltose, 7.5 ppm manganese sulfate pentahydrate, 0.04% calcium chloride dihydrate, and 10 ppm tetracycline; % denotes (w/v)%) was dispensed, and cultured at 32°C at 1,500 rpm for 2 days. Then, the culture supernatant containing the enzyme produced from the bacterial cells was collected by centrifugation and used as an enzyme solution.

### (4) Evaluation of amylase mutant stability in presence of chelating agent

The storage stability of the mutants and parent enzymes shown in Table 2 in the presence of a chelating agent was evaluated. AATCC High Efficiency Standard Reference Detergent WITHOUT Brightener was 10-fold diluted with 100 mM citrate buffer (pH: 5.0), and a culture supernatant 5-fold diluted with ion-exchange water was added thereto, followed by incubation at 50°C for 10 minutes to 1 hour. Then, the resultant was 10-fold diluted with ion-exchange water and used to measure the activity. In addition, for the comparison of stability, a solution obtained by diluting Termamyl 300L (Sigma-Aldrich) with ion-exchange water to 200 ppm was similarly evaluated. The activity value of the sample before the treatment at 50°C was used as the initial activity, the deactivation rate per unit time (h) by the treatment at 50°C was calculated, and the half-life (h) was calculated therefrom. The half-life (h) of each mutant was divided by the half-life (h) of the parent polypeptide or Termamyl to determine the relative stability. Table 3-1 and Table 3-2 show the results. It was shown that all of the mutants had enhanced stability in comparison with the parent polypeptides and Termamyl.

**[Table 3-1]**

| | | Residue number (numbering according tof SEQ ID NO: 1) | | Residue number (numbering according to SEQ ID NO: 2) | | | | | | | | Relative stability (parent enzyme = 1) | Relative stability (Termamyl = 1) |
|---|---|---|---|---|---|---|---|---|---|---|---|---|---|
| Mutant No. | Parent enzyme | 178 | 180 | 178 | 181 | 238 | 239 | 240 | 126 | 129 | 201 | | |
| Parent enzyme | LABM | R | D | G | A | F | S | F | N | V | V | 1 | 1.2 |
| | CspAmy2 | R | T | G | A | F | S | F | N | T | I | 1 | 0.7 |
| | AA560 | R | D | G | G | Y | S | F | N | V | I | 1 | 0.6 |
| | SP722 | R | D | G | A | Y | S | F | N | I | V | 1 | 0.9 |
| | AP1378 | R | T | G | A | Y | S | Y | N | I | I | 1 | 1 |
| no.01 | LABM | Δ | Δ | H | E | F | Q | F | N | v | v | 16.9 | 20.4 |
| no. 02 | CspAmy2 | Δ | Δ | H | E | F | Q | F | N | T | I | 4.5 | 3.3 |
| no.03 | AA560 | Δ | Δ | H | E | F | Q | F | N | V | I | 27 | 17.2 |
| no 04 | SP722 | Δ | Δ | H | E | F | Q | F | N | I | V | 5 | 4.9 |
| no 05 | AP1378 | Δ | Δ | H | E | F | Q | F | N | I | I | 7.6 | 7.5 |
| no.06 | LABM | Δ | Δ | H | E | F | Q | F | Y | I | V | 19.2 | 23.2 |
| no. 07 | CspAmy2 | Δ | Δ | H | E | F | Q | F | Y | I | I | 9.1 | 6.7 |
| no. 08 | AA560 | Δ | Δ | H | E | F | Q | F | Y | I | I | 38.2 | 24.3 |
| no. 09 | SP722 | Δ | Δ | H | E | F | Q | F | Y | I | v | 5.6 | 5.5 |
| no. 10 | AP1378 | Δ | Δ | H | E | F | Q | F | Y | I | I | 9 | 8.9 |
| no. 11 | LABM | Δ | Δ | H | E | F | Q | F | N | V | L | 61.8 | 74.6 |
| no. 12 | CspAmy2 | Δ | Δ | H | E | F | Q | F | N | T | L | 7.9 | 5.7 |
| no 13 | AA560 | Δ | Δ | H | E | F | Q | F | N | V | L | 57.2 | 25.4 |
| no. 14 | SP722 | Δ | Δ | H | E | F | Q | F | N | I | L | 17.2 | 15.6 |
| no. 15 | AP1378 | Δ | Δ | H | E | F | Q | F | N | I | L | 19.7 | 19.7 |
| no 16 | LABM | Δ | Δ | H | E | F | Q | F | N | v | Y | 30.6 | 36.9 |
| no. 17 | CspAmy2 | Δ | Δ | H | E | F | Q | F | N | T | Y | 20.9 | 15.3 |
| no. 18 | AA560 | Δ | Δ | H | E | F | Q | F | N | V | Y | 137.2 | 61 |
| no. 19 | SP722 | Δ | Δ | H | E | F | Q | F | N | I | Y | 36.2 | 32.8 |
| no. 20 | AP1378 | Δ | Δ | H | E | F | Q | F | N | I | Y | 57.1 | 55.8 |

**[Table 3-2]**

| | | Residue number (numbering of SEQ ID NO: 1) | | Residue number (numbering of SEQ ID NO: 2) | | | | | | | | Relative stability (parent enzyme = 1) | Relative stability (Termamyl = 1) |
|---|---|---|---|---|---|---|---|---|---|---|---|---|---|
| Mutant No | Parent enzyme | 178 | 180 | 17 8 | 18 1 | 23 8 | 23 9 | 24 0 | 12 6 | 12 9 | 20 1 | | |
| no. 21 | LABM | Δ | Δ | H | Q | F | Q | F | N | V | V | 17.3 | 20.9 |
| no. 22 | CspAmy 2 | Δ | Δ | H | Q | F | Q | F | N | T | I | 4.9 | 3.6 |
| no. 23 | AA560 | Δ | Δ | H | Q | F | Q | F | N | V | I | 25.4 | 16.2 |
| no. 24 | SP722 | Δ | Δ | H | Q | F | Q | F | N | I | V | 4.7 | 4.6 |
| no. 25 | AP1378 | Δ | Δ | H | Q | F | Q | F | N | I | I | 7.8 | 7.7 |
| no. 26 | LABM | Δ | Δ | H | Q | F | Q | F | Y | I | V | 18.9 | 22.8 |
| no. 27 | CspAmy 2 | Δ | Δ | H | Q | F | Q | F | Y | I | I | 9.2 | 6.8 |
| no. 28 | AA560 | Δ | Δ | H | Q | F | Q | F | Y | I | I | 27.5 | 17.5 |
| no. 29 | SP722 | Δ | Δ | H | Q | F | Q | F | Y | I | V | 4.8 | 4.7 |
| no. 30 | AP1378 | Δ | Δ | H | Q | F | Q | F | Y | I | I | 8.2 | 8.1 |
| no. 31 | LABM | Δ | Δ | H | Q | F | Q | F | N | V | L | 43.2 | 52.1 |
| no. 32 | CspAmy 2 | Δ | Δ | H | Q | F | Q | F | N | T | L | 7.9 | 5.8 |
| no. 33 | AA560 | Δ | Δ | H | Q | F | Q | F | N | V | L | 54.1 | 34.4 |
| no. 34 | SP722 | Δ | Δ | H | Q | F | Q | F | N | I | L | 15.6 | 15.3 |
| no. 35 | AP1378 | Δ | Δ | H | Q | F | Q | F | N | I | L | 83.4 | 82.3 |
| no. 36 | LABM | Δ | Δ | H | Q | F | Q | F | N | V | Y | 30.4 | 36.7 |
| no. 37 | CspAmy 2 | A | Δ | H | Q | F | Q | F | N | T | Y | 20.1 | 14.7 |
| no. 38 | AA560 | Δ | Δ | H | Q | F | Q | F | N | V | Y | 27.8 | 17.7 |
| no. 39 | SP722 | Δ | Δ | H | Q | F | Q | F | N | I | Y | 4.7 | 4.6 |
| no. 40 | AP1378 | Δ | Δ | H | Q | F | Q | F | N | I | Y | 15.7 | 15.5 |

### (5) Evaluation of amylase mutant stability in weak acidic diluted cleaning agent

AATCC High Efficiency Standard Reference Detergent WITHOUT Brightener was 10-fold diluted with 20 mM citrate buffer (pH: 5.0), and a culture supernatant 5-fold diluted with ion-exchange water was added thereto, followed by incubation at 50°C for 30 minutes to 18 hours. Then, the resultant was 10-fold diluted with 50 mM Tris-HCl (pH: 7.5) and used to measure the activity. Thereafter, the relative stability of the mutants was determined in the same manner as in (4). Table 4-1 and Table 4-2 show the results. It was shown that all of the mutants had enhanced stability in comparison with the parent polypeptides and Termamyl.

**[Table 4-1]**

| | | Residue number (numbering according to SEQ ID NO: 1) | | Residue number (numbering according to SEQ ID NO: 2) | | | | | | | | Relative stability (parent enzyme = 1) | Relative stability (Termamyl = 1) |
|---|---|---|---|---|---|---|---|---|---|---|---|---|---|
| Mutant No. | Parent enzyme | 178 | 180 | 178 | 181 | 238 | 239 | 240 | 126 | 129 | 201 | | |
| Parent enzyme | LABM | R | D | G | A | F | S | F | N | V | V | 1 | 2.2 |
| | CspAmy 2 | R | T | G | A | F | S | F | N | T | I | 1 | 0.6 |
| | AA560 | R | D | G | G | Y | S | F | N | V | I | 1 | 0.8 |
| | SP722 | R | D | G | A | Y | S | F | N | I | V | 1 | 0.8 |
| | AP1378 | R | T | G | A | Y | S | Y | N | I | I | 1 | 0.4 |
| no. 01 | LABM | Δ | Δ | H | E | F | Q | F | N | V | V | 6 | 12.1 |
| no. 02 | CspAmy 2 | Δ | Δ | H | E | F | Q | F | N | T | I | 14.9 | 7.6 |
| no. 03 | AA560 | Δ | Δ | H | E | F | Q | F | N | V | I | 13.9 | 10.1 |
| no. 04 | SP722 | Δ | Δ | H | E | F | Q | F | N | I | V | 10.9 | 8.1 |
| no. 05 | AP1378 | Δ | Δ | H | E | F | Q | F | N | I | I | 24.9 | 9.8 |
| no. 06 | LABM | Δ | Δ | H | E | F | Q | F | Y | I | V | 7.6 | 15.2 |
| no. 07 | CspAmy 2 | Δ | Δ | H | E | F | Q | F | Y | I | I | 18.1 | 9.3 |
| no. 08 | AA560 | Δ | Δ | H | E | F | Q | F | Y | I | I | 16.2 | 11.7 |
| no. 09 | SP722 | Δ | Δ | H | E | F | Q | F | Y | I | V | 13.3 | 9.8 |
| no. 10 | AP1378 | Δ | Δ | H | E | F | Q | F | Y | I | I | 29.7 | 11.6 |
| no. 11 | LABM | Δ | Δ | H | E | F | Q | F | N | v | L | 8.8 | 17.6 |
| no. 12 | CspAmy 2 | Δ | Δ | H | E | F | Q | F | N | T | L | 17.4 | 8.9 |
| no. 13 | AA560 | Δ | Δ | H | E | F | Q | F | N | V | L | 16.7 | 12.1 |
| no. **14** | SP722 | Δ | Δ | H | E | F | Q | F | N | I | L | 12.3 | 9.1 |
| no. 15 | AP1378 | Δ | Δ | H | E | F | Q | F | N | I | L | 29.3 | 11.5 |
| no. 16 | LABM | Δ | Δ | H | E | F | Q | F | N | v | Y | 7.2 | 14.4 |
| no. 17 | CspAmy 2 | Δ | Δ | H | E | F | Q | F | N | T | Y | 14.2 | 7.3 |
| no. 18 | AA560 | Δ | Δ | H | E | F | Q | F | N | v | Y | 37.6 | 27.2 |
| no. 19 | SP722 | Δ | Δ | H | E | F | Q | F | N | I | Y | 13.7 | 10.1 |
| no. 20 | AP1378 | Δ | Δ | H | E | F | Q | F | N | I | Y | 97.8 | 38.3 |

**[Table 4-2]**

| | | Residue number (numbering according to SEQ ID NO: 1) | | Residue number (numbering according to SEQ ID NO: 2) | | | | | | | | Relative stability (parent enzyme = 1) | Relative stability (Termamyl = 1) |
|---|---|---|---|---|---|---|---|---|---|---|---|---|---|
| Mutant No | Parent enzyme | 178 | 180 | 178 | 181 | 238 | 239 | 240 | 126 | 129 | 201 | | |
| no. 21 | LABM | Δ | Δ | H | Q | F | Q | F | N | V | V | 3 | 6.1 |
| no. 22 | CspAmy 2 | Δ | Δ | H | Q | F | Q | F | N | T | I | 12.1 | 6.2 |
| no. 23 | AA560 | Δ | Δ | H | Q | F | Q | F | N | V | I | 10.8 | 7.9 |
| no. 24 | SP722 | Δ | Δ | H | Q | F | Q | F | N | I | V | 7.2 | 5.4 |
| no. 25 | AP1378 | Δ | Δ | H | Q | F | Q | F | N | I | I | 20.3 | 8 |
| no. 26 | LABM | Δ | Δ | H | Q | F | Q | F | Y | I | V | 3.1 | 6.2 |
| no. 27 | CspAmy 2 | Δ | Δ | H | Q | F | Q | F | Y | I | I | 27.2 | 13.9 |
| no. 28 | AA560 | Δ | Δ | H | Q | F | Q | F | Y | I | I | 16.3 | 11.8 |
| no. 29 | SP722 | Δ | Δ | H | Q | F | Q | F | Y | I | V | 9.5 | 2.8 |
| no. 30 | AP1378 | Δ | Δ | H | Q | F | Q | F | Y | I | I | 31.5 | 12.4 |
| no. 31 | LABM | Δ | Δ | H | Q | F | Q | F | N | V | L | 6.3 | 12.6 |
| no. 32 | CspAmy 2 | Δ | Δ | H | Q | F | Q | F | N | T | L | 22.6 | 11.5 |
| no. 33 | AA560 | Δ | Δ | H | Q | F | Q | F | N | V | L | 15.4 | 11.2 |
| no. 34 | SP722 | Δ | Δ | H | Q | F | Q | F | N | I | L | 18.4 | 13.7 |
| no. 35 | AP1378 | Δ | Δ | H | Q | F | Q | F | N | I | L | 89.5 | 35.2 |
| no. 36 | LABM | Δ | Δ | H | Q | F | Q | F | N | V | Y | 9.7 | 19.7 |
| no. 37 | CspAmy 2 | Δ | Δ | H | Q | F | Q | F | N | T | Y | 19.1 | 9.8 |
| no. 38 | AA560 | Δ | Δ | H | Q | F | Q | F | N | V | Y | 11.9 | 8.6 |
| no. 39 | SP722 | Δ | Δ | H | Q | F | Q | F | N | I | Y | 8 | 6 |
| no. 40 | AP1378 | Δ | Δ | H | Q | F | Q | F | N | I | Y | 37.7 | 14.8 |

## Claims

1. α-Amylase mutants A1, B1, C1, D1, and E1 derived from the following parent amylases: an α-amylase A having at least 90% sequence identity to the amino acid sequence of SEQ ID NO: 4; an α-amylase B having at least 90% sequence identity to the amino acid sequence of SEQ ID NO: 6; an α-amylase C having at least 90% sequence identity to the amino acid sequence of SEQ ID NO: 8; an α-amylase D having at least 90% sequence identity to the amino acid sequence of SEQ ID NO: 10; and an α-amylase E having at least 90% sequence identity to the amino acid sequence of SEQ ID NO: 12, respectively, wherein the mutants A1 to E1 each comprises deletion of amino acid residues at two positions selected from the group consisting of positions corresponding to positions 178 to 181 according to the numbering of SEQ ID NO: 1, and substitution of amino acid residues at three or more positions selected from the group consisting of positions corresponding to positions 178, 181, 238, 239, 240, 126, 129, and 201 according to the numbering of SEQ ID NO: 2, the substitution including at least the following a) to e):
a) in A1, substitution of an amino acid residue at a position corresponding to position 178 with H, substitution of an amino acid residue at a position corresponding to position 181 with E or Q, and substitution of an amino acid residue at a position corresponding to position 239 with Q;
b) in B1, substitution of an amino acid residue at a position corresponding to position 178 with H, substitution of an amino acid residue at a position corresponding to position 181 with E or Q, and substitution of an amino acid residue at a position corresponding to position 239 with Q;
c) in C1, substitution at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q;
d) in D1, substitution at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q; and
e) in E1, substitution at three or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F.

2. The α-amylase mutant according to claim 1, wherein
the substitution in C1 is substitution at four positions including substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q according to the numbering of SEQ ID NO: 2;
the substitution in D1 is substitution at four positions including substitution of an amino acid residue at a position corresponding to position 178 with H, substitution of an amino acid residue at a position corresponding to position 181 with E or Q, substitution of an amino acid residue at a position corresponding to position 238 with F, and substitution of an amino acid residue at a position corresponding to position 239 with Q according to the numbering of SEQ ID NO: 2; and
the substitution in E1 is substitution at four or more positions selected from the group consisting of substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F according to the numbering of SEQ ID NO: 2.

3. The α-amylase mutant according to claim 2, wherein the substitution in E1 is substitution at five positions including substitution of an amino acid residue at a position corresponding to position 178 with H, substitution of an amino acid residue at a position corresponding to position 181 with E or Q, substitution of an amino acid residue at a position corresponding to position 238 with F, substitution of an amino acid residue at a position corresponding to position 239 with Q, and substitution of an amino acid residue at a position corresponding to position 240 with F according to the numbering of SEQ ID NO: 2.

4. The α-amylase mutant according to any one of claims 1 to 3, wherein the mutant further comprises, in a) to e) above, substitution of amino acid residue(s) at one or more positions selected from the group consisting of positions corresponding to positions 126, 129, and 201 according to the numbering of SEQ ID NO: 2.

5. The α-amylase mutant according to claim 4, wherein the amino acid residues after substitution at positions corresponding to positions 126, 129, and 201 in the α-amylase are 126Y, 129I, and 201L/Y, respectively.

6. A polynucleotide encoding the mutant according to any one of claims 1 to 5.

7. A vector or DNA fragment comprising the polynucleotide according to claim 6.

8. A transformed cell comprising the vector or DNA fragment according to claim 7.

9. The transformed cell according to claim 8, wherein the transformed cell is a microorganism.

10. A cleaning composition comprising the mutant according to any one of claims 1 to 5.

11. The cleaning composition according to claim 10, wherein the cleaning composition is a clothing cleaning agent or a dishwashing cleaning agent.

12. The cleaning composition according to claim 11, wherein the cleaning composition is a powder or a liquid.

13. The cleansing composition according to claim 11 or 12, wherein the cleaning composition is weakly acidic.

14. The cleansing composition according to any one of claims 11 to 13, wherein the cleaning composition comprises a chelating agent.
